# DEMANDE DE BREVET EUROPEEN

(11) **EP 0 895 979 A1**
(43) Date de publication de la demande: **10.02.1999**
(21) Numéro de dépôt: 98401601.4
(22) Date de dépôt: 26.06.1998
(51) Int. Cl.: C07C 31/24, A61K 7/06, C11D 1/62

(54) **Ammonium Quaternaires d'alkylamido alditiols, procédé de préparation, compositions les contenant et utilisations**

(30) Priorité: 08.08.1997 FR 9710215
(71) Demandeur: CECA S.A., 92800 Puteaux (FR)
(72) Inventeur: Petit, Serge, 74540 Cusy (FR)
(74) Mandataire: Haicour, Philippe

(57) **Abrégé**

L'invention a pour objet des composés de formule 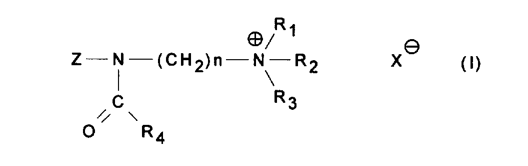 dans laquelle
Z représente un radical aldityle ou glycosylaldityle
R₁, R₂ et R₃, identiques ou différents, représentent un radical alkyle en C₁-C₄ ou hydroxyalkyle en C₂-C₄,
R₄-C = O est un radical acyle aliphatique, linéaire ou ramifié, saturé ou insaturé en C₂-C₂₂,
X représente un atome d'halogène ou un groupe de formule SO₃-O-R₃, R₃ étant nécessairement un radical alkyle tel que défini ci-avant,
n est un nombre entier de 1 à 4.

Elle concerne également un procédé de préparation de ces composés et leurs utilisations, notamment dans des compositions cosmétiques et capillaires.

## Description

La présente invention concerne des ammonium quaternaires d'alkylamido alditols, des compositions les contenant, leur procédé de préparation et leurs utilisations.

Les surfactants cationiques en général ont la capacité de s'adsorber sur les surfaces solides, en particulier sur celles qui possèdent une charge globale négative. Ce groupe de surfactants trouve des applications dans de nombreux secteurs de l'industrie chimique, en particulier dans le domaine des cosmétiques. Leurs propriétés sont multiples : dispersion de pigments, agent de floculation ou de précipitation, adjuvant de mouillage ou de broyage, germicide à l'égard des micro-organismes du type Gram positif. En outre, ces surfactants développent des propriétés émulsifiantes quand ils sont associés à des tensioactifs non ioniques, ou à des tensioactifs anioniques lorsqu'ils sont sous la forme de "sels neutres".

Dans le domaine cosmétique et capillaire, la principale fonction des surfactants cationiques est le conditionnement de la peau et des cheveux. Ils pénètrent la chevelure humide et agissent au niveau des interactions entre les cheveux, réduisant ainsi le problème du démêlage au moment du séchage et du peignage. Par ailleurs, ils améliorent la texture et la douceur du cheveu, réduisent les effets dus à l'électricité statique en neutralisant les charges anioniques apparentes, évitent la formation des fourches, contribuent à un meilleur état du cheveu et préviennent les dommages résultant des agressions externes. Enfin, ils permettent d'assurer la compatibilité du cheveu avec les autres constituants présents dans les compositions tels que les alcools gras, les esters d'acides gras et les parfums.

Les surfactants cationiques ont cependant l'inconvénient d'être irritants pour la peau et les yeux, voire même de provoquer des dommages irréversibles. Il est nécessaire de les employer avec précaution en tenant compte à la fois de leur nature chimique et de la concentration à laquelle ils sont présents dans les compositions cosmétiques ou capillaires.

Le besoin est donc grand de disposer de nouveaux tensioactifs cationiques susceptibles de présenter un faible pouvoir irritant.

Il a maintenant été trouvé de nouveaux tensioactifs cationiques.

Les tensioactifs selon l'invention appartiennent au groupe des ammonium quaternaires d'alkylamido alditols de formule : 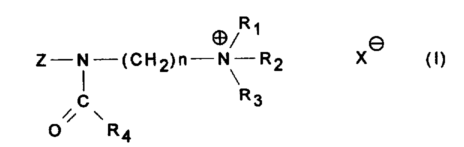 dans laquelle :
Z représente un radical aldityle ou glycosylaldityle,
R₁, R₂ et R₃, identiques ou différents, représentent un radical alkyle en C₁-C₄ ou hydroxyalkyle en C₂-C₄,
R₄-C = O est un radical acyle aliphatique, linéaire ou ramifié, saturé ou insaturé en C₂-C₂₂,
X représente un atome d'halogène ou un groupe de formule SO₃-O-R₃, R₃ étant nécessairement un radical alkyle tel que défini ci-avant,
n est un nombre entier de 1 à 4.

Les composés préférés selon l'invention sont les composés de formule (I) dans laquelle :
Z représente un radical pentityle, hexityle, glycosylpentityle ou glycosylhexityle renfermant 2 à 20 motifs de monosaccharides,
R₁, R₂ et R₃, identiques ou différents, représentent un radical méthyle, éthyle ou propyle, R₁, R₂ et R₃ étant préférentiellement identiques,
R₄-C = O est un radical acyle de chaîne grasse en C₇-C₁₈,
X représente Cl, Br, I ou SO₃-O-R₃, R₃ ayant la signification donnée ci-avant,
n est égal à 3.

A titre d'exemple de résidus pentityles, on peut citer les résidus de l'arabinose, du ribose et du xylose, et pour les résidus hexityles, les résidus du glucose, du galactose, du mannose et du talose. De manière avantageuse, le résidu est un hexityle, et plus particulièrement du glucose (radical sorbityle).

Parmi les résidus glycosylpentityles et glycosylhexityles, on préfère les résidus de disaccharides tels que le lactose ou le maltose, ou de trisaccharides tels que le maltotriose. De manière avantageuse, le résidu est un résidu de disaccharide, et plus particulièrement du lactose (radical lactityle) ou du maltose (radical maltityle).

Des exemples de radicaux R₄-C = O sont les restes acyles des acides caprylique, caprique, undécylénique, laurique, palmitique, stéarique ou oléique. Ces acides peuvent par exemple être obtenus à partir d'huiles de coco, d'huiles de suif non hydrogéné ou de suif partiellement ou totalement hydrogéné, et de composés comparables.

Le résidu d'acide gras est souvent un mélange d'au moins deux groupes acyles, par exemple des coupes C₁₂/C₁₄, C₁₆/C₁₈ ou C₁₂ à C₁₈.

Le procédé de préparation des composés de formule (I), qui constitue également un objet de la présente invention, consiste à faire réagir le composé de formule : 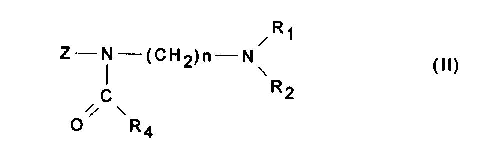 dans laquelle Z, R₁, R₂, R₄ et n ont la signification donnée précédemment,
- avec un agent de quaternisation de formule :

   R₃X (III)

   dans laquelle R₃ et X ont la signification donnée précédemment.

La réaction est effectuée en mettant en contact le composé de formule (ll) avec l'agent de quaternisation dans un rapport molaire de 1:0,95 à 1:3, de préférence 1:1 à 1:2.

Bien que la réaction puisse être effectuée en l'absence de solvant, on préfère utiliser un solvant, de préférence anhydre, choisi parmi les alcools tels que le méthanol, l'éthanol, le propanol, l'isopropanol, le butanol ou l'isobutanol, les éther-oxydes tels que le tétrahydrofurane ou l'éther diméthylique de l'éthylène glycol, et les hydrocarbures halogénés tels que le dichlorométhane, le dichloroéthane ou le chloroforme. On préfère les hydrocarbures halogénés.

La quantité de solvant est choisi de telle sorte que, au moment de la mise en contact des réactifs, le composé de formule (II) se trouve à une concentration variant de 3 à 99,9 % en masse, de préférence 5 à 30 %, calculée sur la masse de la solution ou de la dispersion.

La réaction est généralement conduite à une température comprise entre 0 et 130°C, de préférence 30 à 80°C, et à la pression atmosphérique jusqu'à la consommation complète des réactifs ou, le cas échéant, du réactif en défaut.

Le milieu réactionnel contenant le composé de formule (I) peut, le cas échéant, être traité avec de l'eau afin d'hydrolyser l'excès d'agent de quaternisation. Le milieu réactionnel peut être concentré sous une pression comprise entre 0,013 et 101,325 kPa, de préférence 0,013 et 4 kPa. Le résidu ainsi obtenu peut être repris avec de l'eau permutée et lyophilisé, ce qui a pour effet d'améliorer l'aspect du solide résultant.

Les composés de formule (II) nécessaires pour la synthèse des composés selon l'invention peuvent être obtenus avantageusement par réaction du composé de formule : 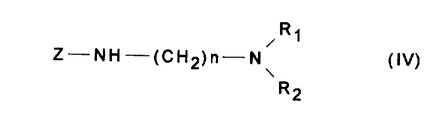 avec un agent acylant de formule :

R₄―CO―R₅ (V)

dans lesquelles :
Z, R₁, R₂, R₄ et n ont la signification donnée précédemment et,
R₅ représente un atome d'halogène, de préférence Cl, un groupe O-CO-R₆ dans lequel R₆ est une chaîne hydrocarbonée linéaire ou ramifiée, saturée ou insaturée en C₁-C₂₁, de préférence C₆-C₁₇, ou un groupe O-R₇ dans lequel R₇ est un radical alkyle en C₁-C₄ ou diglycéryle dont les résidus acyles, identiques ou différents, renferment 2 à 22 atomes de carbone, de préférence 7 à 18 atomes.

Lorsque l'agent acylant est un anhydride d'acide, on préfère qu'il soit symétrique (R₄ = R₆).

La réaction d'acylation est généralement effectuée en mettant en contact le composé de formule (IV) avec l'agent acylant dans un rapport molaire de 1:0,95 à 1:4, de préférence 1:1 à 1:3, en l'absence de solvant ou en présence d'un solvant tel que l'eau, un alcool à chaîne courte par exemple le méthanol, l'éthanol ou l'isopropanol, ou un mélange hydroalcoolique d'un ou plusieurs de ces alcools. On préfère utiliser un mélange hydroalcoolique.

Lorsque l'agent acylant est un anhydride ou un halogénure d'acide, la réaction est en général menée à une température comprise entre -10 et +60°C, de préférence -5 et +30°C, et à la pression atmosphérique. Lorsque l'agent acylant est un ester, on opère généralement entre 60 et 150°C, de préférence 70 et 130°C, et à une pression pouvant varier de 0,013 à 101,325 kPa, de préférence 0,013 à 10 kPa.

Le pH de la réaction est généralement maintenu entre 7 et 11, de préférence 7 et 10, ceci pendant toute la durée de la réaction. On peut pour cela utiliser une base choisie parmi les hydroxydes de formule M(OH)_{X} dans laquelle M représente un métal alcalin ou alcalino-terreux et x est la valence du métal, ou un précurseur d'une telle base, par exemple les carbonates ou les hydrogénocarbonates, notamment d'un métal alcalin tel que le sodium ou le potassium, et les alcanoates de métal alcalin tels que le méthanoate ou l'éthanoate de sodium.

A l'issue de la réaction, c'est-à-dire après consommation complète des réactifs ou, le cas échéant, du réactif en défaut, on récupère le milieu réactionnel contenant le composé de formule (II) qui peut être utilisé tel quel ou subir une étape de purification, par exemple par chromatographie sur de la silice.

Quant aux composés de formule (IV), ils peuvent être avantageusement obtenus par réaction d'un monosaccharide ou d'un oligosaccharide permettant l'obtention du résidu Z tel que défini précédemment avec une amine de formule : 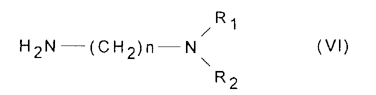 dans lesquelles Z, R₁, R₂ et n ont la signification donnée précédemment ;
- dans l'eau ou un mélange hydroalcoolique et en présence d'un catalyseur d'hydrogénation.

Les composés de formule (I) selon l'invention peuvent être utilisés pour préparer des compositions ayant des propriétés tensioactives, notamment des compositions cosmétiques et capillaires.

Les compositions selon l'invention comprennent une base détergente constituée pour tout ou partie d'un ou plusieurs composés de formule (I), d'un véhicule et, le cas échéant, d'additifs.

La base détergente représente généralement 0,1 à 50 % en poids, de préférence 1 à 30 %, de la composition. Elle peut être constituée d'un ou plusieurs composés de formule (I) ou d'un mélange d'un ou plusieurs composés de formule (I) et d'un ou plusieurs tensioactifs connus de l'homme du métier tels que les tensioactifs anioniques, cationiques, non ioniques et amphotères. Les composés selon l'invention représentent généralement 1 à 100 % en poids de la base détergente, et de préférence 10 à 50 %.

Le véhicule est généralement l'eau ou un solvant organique compatible avec une application topique. A titre d'exemple d'un tel solvant, on peut citer l'acétone, l'alcool isopropylique, les triglycérides d'acides gras en C₆-C₂₄, les éthers de glycol tels que les éthers d'alkyle en C₂-C₄ de monoalkylène ou dialkylène glycol, les esters de polyalkylène glycol et d'acides en C₂-C₄, et les silicones volatils.

Les compositions selon l'invention peuvent contenir, en outre, des additifs tels que des corps gras (par exemple des huiles naturelles ou synthétiques), des épaississants ou des gélifiants, des matières actives (par exemple des agents hydratants), des antioxydants, des conservateurs, des parfums et des colorants.

Les compositions selon l'invention peuvent se présenter sous différentes formes. Par exemple, elles peuvent avoir l'aspect d'une lotion moussante ou non moussante, d'une émulsion de consistance liquide ou semi-liquide telle qu'un lait obtenu par dispersion d'une phase grasse dans une phase aqueuse ou inversement, d'une suspension ou d'une émulsion de consistance molle telle qu'une crème ou une pommade, d'un gel ou encore d'une préparation solide telle qu'un "stick", un pain de nettoyage, un tampon imprégné ou un masque hydratant.

Les compositions cosmétiques peuvent être un bain moussant, un gel douche ou un savon liquide doux.

Les compositions capillaires peuvent être un shampooing, par exemple un shampooing doux à usage fréquent, ou un après-shampooing ayant notamment la propriété de conditionner le cheveu.

Les composés selon l'invention peuvent également être employés comme adjuvants de mouillage ou de broyage, agents de floculation ou de précipitation, ou encore comme dispersants de pigments.

Les exemples qui suivent permettent d'illustrer l'invention. Dans ces exemples, on utilise les méthodes d'analyse ci-après.
- Le R_{F} est mesuré par chromatographie sur couche mince de silice (épaisseur du film : 200 µm ; taille des particules : 5-10 µm). Le solvant de migration est un mélange méthanol/ammoniaque à 25 % 20/1 (v/v). Les spots de migration sont révélés par pulvérisation d'une solution d'acide phosphomolybdique à 25 % en poids dans l'éthanol et chauffage.
- Résonance magnétique nucléaire (RMN ¹³C)
   Les spectres sont réalisés à 50 MHz dans CDCl₃, la référence interne étant le tétraméthylsilane. Les déplacements chimiques sont exprimés en ppm.
- Spectroscopie infrarouge (IR)
   Les spectres sont réalisés sur une pastille de KBr (1 %) ou sur film. Les valeurs sont exprimées en nombre d'onde par cm.

### EXEMPLE 1

### a) Préparation de l'alkylamido alditol de formule :

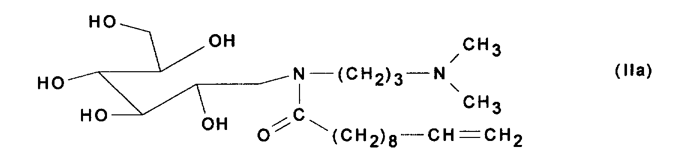

Dans un ballon tricol de 250 ml surmonté d'un réfrigérant et équipé d'un thermomètre, d'une ampoule de coulée et d'une agitation magnétique, on introduit 5,4 g (0,02 mole) de N-(3-diméthylaminopropyl) glucamine, 60 ml d'un mélange eau/éthanol (1/3 ; v/v) et 5,3 g (0,05 mole) de carbonate de sodium (Réf. 22,232-1 ; ALDRICH).

La N-(3-diméthylaminopropyl) glucamine est préparée par réaction stoechiométrique du glucose et de 3-diméthylaminopropylamine dans le méthanol (45°C; 4 heures) et réduction du N-glucoside obtenu par le nickel de Raney (60°C; 12 heures; 3000 kPa).

Le milieu réactionnel est refroidi à 0°C et on y ajoute en une heure 4,16 g (0,04 mole) de chlorure d'acide undécylénique (Réf. 16,166-7 ; ALDRICH).

Le milieu réactionnel est maintenu à 0°C pendant une heure, et à 20°C pendant 5 heures sous une forte agitation. Après filtration (verre fritté n°3), le filtrat est récupéré et évaporé sous vide (20°C ; 2,2 kPa). Le résidu ainsi obtenu est chromatographié sur une colonne de silice (éluant : méthanol/acétate d'éthyle 1/1 ; v/v, puis méthanol/ammoniaque à 25 % 20/1 ; v/v).

On récupère le composé de formule (IIa) avec un rendement de 38 % calculé sur la base de la glucamine de départ. Ce composé présente les caractéristiques suivantes :
R_{F} : 0,30
RMN¹³C :
Partie sucre : 49,91 ; 72,73 ; 71,91 ; 73,37 ; 70,29 ; 64,01.
Carbonyle : 175,28 ; 174,72.
Autres : 47,74 ; 23,85-23,86 ; 59,96-56,52 ; 45,48-45,08 ; 33,90-33,55 ; 26,92-29,03-29,23-29,29-29,55-29,63-29,75 ; 25,05 ; 139,28-139,21 ; 114,29-114,24.
IR : 3371 ; 2928 ; 2857 ; 1620.

### b) Préparation du dérivé quaternaire de l'alkylamido alditol de formule :

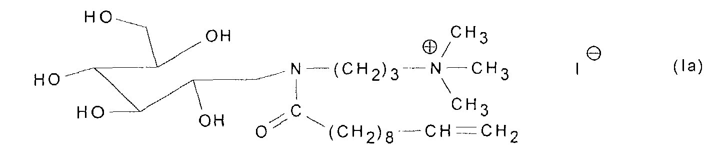

Dans un ballon tricol de 100 ml surmonté d'un réfrigérant muni d'une garde (CaCl₂) et équipé d'un thermomètre et d'une agitation magnétique, on introduit, à 20°C, 4,33 g (10 mmoles) du composé (IIa) de l'étape a-, 50 ml de dichlorométhane anhydre et, goutte à goutte, 2,28 g (16 mmoles) d'iodométhane (Réf. I-850-7 ; ALDRICH).

Après 4 heures de réaction à 40°C, le mélange réactionnel est refroidi à 20°C et on y ajoute 30 ml d'eau. Après évaporation du solvant sous pression réduite (20°C ; 2,4 kPa), le résidu est repris avec 30 ml de méthanol et concentré à nouveau dans les conditions précitées. L'opération est renouvelée 3 fois.

On récupère le composé de formule (la) avec un rendement de 93 % calculé sur la base de l'alkylamido alditol de départ.

Ce composé présente les caractéristiques suivantes :
RMN¹³C :
Partie sucre : 49,21-50,09 ; 71,68-71,60-70,89-70,22 ; 72,63 ; 70,22-69,70 ; 63,52-62,93.
Carbonyle : 173,08-172,84.
Autres : 45,88-45,68 ; 21,19-22,27 ; 42,90 ; 52,45 ; 33,41 ; 25,21-28,75-29,01-29,08-29,10-29,21 ; 32,54 ; 25,20 ; 139,01 ; 114,84.

### EXEMPLE 2

### a) Préparation de l'alkylamido alditol de formule :

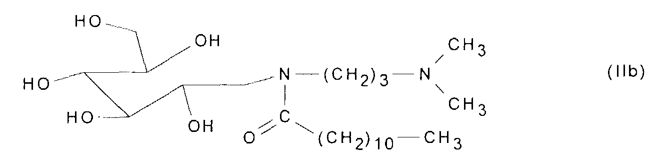

On procède dans les conditions de l'exemple 1 modifié en ce que l'on utilise 8,75 g (0,04 mole) de chlorure d'acide dodécanoïque (Réf. 15,693-0 ; ALDRICH).

A l'issue de la réaction, on récupère le composé de formule (llb) avec un rendement de 57 % calculé sur la base de la glucamine de départ.

Ce composé présente les caractéristiques suivantes :
R_{F} : 0,36
RMN¹³C :
Partie sucre : 49,77 ; 72,59 ; 71,81 ; 73,28 ; 70,14 ; 63,85
Carbonyle : 175,15-175,74.
Autres : 47,57 ; 22,67 ; 56,33 ; 45,27-44,79 ; 33,10-33,42 ; 25,44 ; 26,70-27,23-29,32-29,44-29,53-29,77-31,90 ; 24,91 ; 14,14.
IR : 3368 ; 2855 ; 2927 ; 2822 ; 2782 ; 1620.

### b) Préparation du dérivé quaternaire de l'alkylamido alditol de formule :

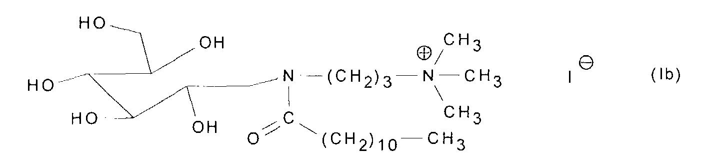

On procède dans les conditions de l'exemple 1 modifié en ce que l'on utilise 4,5 g (10 mmoles) du composé (IIb) de l'étape a- ci-avant.

On récupère le composé de formule (Ib) avec un rendement de 92 % calculé sur la base de l'alkylamido alditol de départ.

Ce composé présente les caractéristiques suivantes :
RMN¹³C :
Partie sucre : 50,03-49,15 ; 71,56 ; 70,11 ; 72,56 ; 69,60 ; 63,50-63,37.
Carbonyle : 173,10-172,88.
Autres : 47,59 ; 21,15-22,24 ; 42,83 ; 52,38 ; 32,47-32,40 ; 23,37 ; 25,00-25,14-28,50-28,88-28,95-29,06-29,20-29,33-31,44 ; 22,54 ; 14,09.

### EXEMPLE 3

### a) Préparation de l'alkylamido alditol de formule :

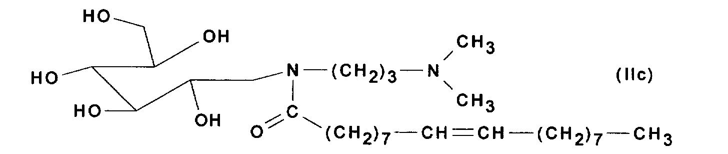

On procède dans les conditions de l'exemple 1 modifié en ce que l'on utilise 12 g (0,04 mole) de chlorure d'acide cis-9-octadécénoïque (Réf. 36,785-0 ; ALDRICH) préalablement distillé (point d'ébullition : 193°C/0,53 kPa).

On récupère le composé de formule (IIc) avec un rendement de 44 % calculé sur la base de la glucamine de départ.

Ce composé présente les caractéristiques suivantes :
R_{F} : 0,38
RMN¹³C :
Partie sucre : 49,77 ; 72,62 ; 71,87 ; 73,30 ; 70,25 ; 63,91.
Carbonyle : 175,15-174,74.
Autres : 47,57 ; 22,72 ; 56,42 ; 45,27-44,59 ; 33,42-33,10 ; 26,70-27,23-29,32-29,45-29,53-29,77-31,90 ; 25,45 ; 129,99-129,90-129,78-129,68 ; 24,91 ; 24,20 ; 14,14.
IR : 3370 ; 2928 ; 2855 ; 1620

### b) Préparation du dérivé quaternaire de l'alkylamido alditol de formule :

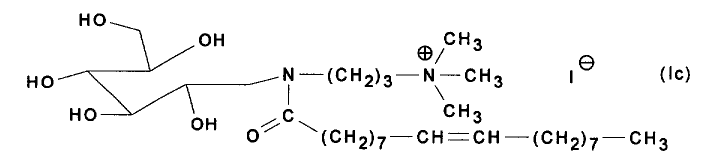

On procède dans les conditions de l'exemple 1 modifié en ce que l'on utilise 5,3 g (10 mmoles) du composé (IIc) de l'étape a- ci-avant.

On récupère le composé de formule (Ic) avec un rendement de 91 % calculé sur la base de l'alkylamido alditol de départ.

Ce composé présente les caractéristiques suivantes :
RMN¹³C :
Partie sucre : 49,72-49,48 ; 71,62-70,30-70,19-69,99 ; 72,63 ; 69,69 ; 63,14.
Carbonyle : 173,08.
Autres : 45,60 ; 20,85-21,94 ; 42,54 ; 52,08 ; 32,17 ; 26,41-26,50-28,43-28,53-28,68-28,79-28,94-29,04-31,11 ; 24,82 ; 129,92-129,82-130,22-129,77 ; 24,82 ; 24,70 ; 14,14.

## Revendications

1. Composés de formule : 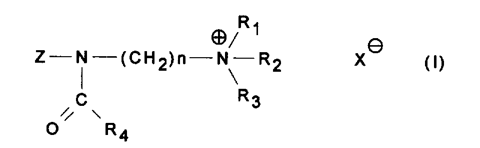 dans laquelle :
Z représente un radical aldityle ou glycosylaldityle,
R₁, R₂ et R₃, identiques ou différents, représentent un radical alkyle en C₁-C₄ ou hydroxyalkyle en C₂-C₄,
R₄-C = O est un radical acyle aliphatique, linéaire ou ramifié, saturé ou insaturé, en C₂-C₂₂,
X représente un atome d'halogène ou un groupe de formule SO₃-O-R₃, R₃ étant nécessairement un radical alkyle tel que défini ci-avant,
n est un nombre entier de 1 à 4.

2. Composés selon la revendication 1, pour lesquels
Z représente un radical pentityle, hexityle, glycosylpentityle ou glycosylhexityle renfermant 2 à 20 motifs de monosaccharides,
R₁, R₂ et R₃, identiques ou différents, représentent un radical méthyle, éthyle ou propyle,
R₄-C = O est un radical acyle de chaîne grasse en C₇-C₁₈,
X représente Cl, Br, I ou SO₃-O-R₃, R₃ ayant la signification donnée ci-avant,
n est égal à 3.

3. Composés selon la revendication 2, pour lesquels R₁, R₂ et R₃ sont identiques.

4. Composés selon l'une des revendications 2 ou 3, pour lesquels Z représente un radical sorbityle, lactityle et maltityle.

5. Procédé de préparation des composés selon les revendications 1 à 4, qui consiste à faire réagir le composé de formule 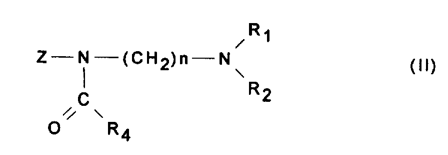 dans laquelle Z, R₁, R₂, R₄ et n ont la signification donnée précédemment,
- avec un agent de quaternisation de formule :
R₃X (III)
dans laquelle R₃ et X ont la signification donnée précédemment.

6. Procédé selon la revendication 5, dans lequel la réaction est effectuée dans un solvant choisi parmi les alcools, les éther-oxydes et les hydrocarbures halogénés.

7. Procédé selon la revendication 6, dans lequel la quantité de solvant est telle que la concentration du composé de formule (II) est comprise entre 3 et 99,9 % en masse de la masse de la dispersion ou de la solution.

8. Procédé selon l'une des revendications 5 à 7, dans lequel le rapport molaire du composé de formule (II) à l'agent de quaternisation est compris entre 1:0,95 et 1:3.

9. Procédé selon l'une des revendications 1 à 8, dans lequel la température est comprise entre 0 et 130°C.

10. Composition cosmétique ou capillaire comprenant un ou plusieurs composés selon l'une des revendications 1 à 4, un véhicule et, le cas échéant des additifs.

11. Composition selon la revendication 10, dans laquelle le ou les composés représentent 0,1 à 50 % en poids de la composition.

12. Utilisation des composés selon l'une des revendications 1 à 4 comme agents de conditionnement du cheveu.

13. Utilisation des composés selon l'une des revendications 1 à 4 comme adjuvants de mouillage ou de broyage.

14. Utilisation des composés selon l'une des revendications 1 à 4 comme agents de floculation ou de précipitation.

15. Utilisation des composés selon l'une des revendications 1 à 4 comme dispersants de pigments.
